# EUROPÄISCHE PATENTSCHRIFT

(11) **EP 1 754 509 B1**
(45) Veröffentlichungstag und Bekanntmachung des Hinweises auf die Patenterteilung: **10.06.2009**
(21) Anmeldenummer: 06014778.2
(22) Anmeldetag: 15.07.2006
(51) Int. Cl.: A61N 1/36, A61F 11/04, A61B 5/053

(54) **Implantat zum Einführen in Hohlkörper**
Implant for the insertion into a hollow body
Implant pour insertion dans un corps creux

(30) Priorität: 18.08.2005 DE 102005039455
(43) Veröffentlichungstag der Anmeldung: 21.02.2007
(73) Patentinhaber: Forschungszentrum Jülich GmbH, 52425 Jülich (DE)
(72) Erfinder: Bousack, Herbert, 52080 Aachen (DE); Zhang, Yi, 52428 Jülich (DE); Otto, Ralph, 4721 Kelmis (BE); Offenhäusser, Andreas, 4731 Eynatten (BE)

(56) Entgegenhaltungen:
- JP-A- 60 103 954
- US-A- 4 447 206
- US-A- 5 112 224
- US-A- 6 070 105
- US-A1- 2003 139 781
- US-A1- 2004 138 714

## Beschreibung

Die Erfindung betrifft ein Implantat, eine Anordnung aus einem Implantat und einem dieses umgebenden Hohlkörper sowie ein Verfahren zur Herstellung dieser Anordnung.

### Stand der Technik

Bei hochgradig Schwerhörigen oder völlig tauben Patienten kann durch eine Versorgung mit einem Cochleaimplantat ein prinzipieller Höreindruck erreicht werden. Das eigentliche Cochleaimplantat besteht im Wesentlichen aus einem Empfänger der akustischen Signale und der Stimulationselektrode, bei der in einem Silikonträger eingebettet beispielsweise 12 Elektrodenpaare über eine Distanz von beispielsweise 25 mm verteilt sind. Die Stimulationselektrode wird durch eine in den Schädel eingebrachte Bohrung in die Cochlea eingeführt und stimuliert dort die Hörnerven direkt elektrisch. Die operative Einführung und Positionierung der Stimulationselektrode in der Cochlea ist sehr schwierig. Speziell ist es nicht möglich, während des Eingriffs den Abstand des Endes des Implantats zu den Wandungen der Cochlea zu bestimmen. Auch gibt es außer einem mechanischen Endanschlag keine Rückmeldung darüber, wann das Ende des Implantats das Ende der Cochlea erreicht. Zur Erleichterung gibt es verschiedene Operationstechniken, z. B. mit einem zusätzlich eingeführten Elektrodenpositionierer. Nachteilig benötigen diese zusätzlich eingeführten Hilfsmittel Raum, der in der beengten Cochlea sehr knapp ist, und stellen außerdem ein zusätzliches Infektionsrisiko dar. Es besteht daher ein Bedürfnis, beim Einführen des Implantats den Abstand zwischen dem Ende des Implantats und den Wandungen beziehungsweise dem Ende der Cochlea online zu überwachen.

Sensoren zur Messung des Abstands sind in den verschiedensten Ausführungsformen und Wirkprinzipien bekannt, z. B. kapazitiv, optisch, per Infrarot oder Ultraschall. Für das vorliegende Problem der Integration in eine Cochleaelektrode und den Einsatz im menschlichen Körper sind diese Sensoren jedoch nicht geeignet, z. B. aus Gründen der Baugröße, der Energieversorgung oder der Energiedissipation.

Das US-Patent 6,552,667 offenbart ein Verfahren, mit dem der Abstand zwischen zwei Körpern über einen auf dem ersten Körper aufgebrachten Sensor gemessen werden kann. Nachteilig ist dieser Sensor ein aus zwei Platten bestehender Kondensator und benötigt daher vergleichsweise viel Einbauraum. Zudem funktioniert das Verfahren nur, wenn das zweite Teil elektrisch leitend und geerdet ist.

Aus dem US-Patent 6,509,744 ist ein Verfahren zur Messung des Abstands zweier Körper bekannt, bei dem eine auf dem ersten Körper aufgebrachte Sensorelektrode und der zweite Körper einen Messkondensator bilden. Der Abstand der beiden Körper wird über Veränderungen der Impedanz dieses Messkondensators bestimmt. Nachteilig muss der zweite Körper elektrisch leitend sein und mit der Messanordnung kontaktiert werden, so dass auch dieses Verfahren nicht dazu geeignet ist, das Einführen eines Cochleaimplantats online zu überwachen.

Ein Implantat nach dem Oberbegriff des Anspruchs 1 ist aus dem US- Patent 4,447,206 bekannt.

### Aufgabe und Lösung

Aufgabe der Erfindung ist es, einen Träger zur Verfügung zu stellen, der in verbesserter Weise gegenüber dem Stand der Technik in einen Hohlraum einführbar ist. Aufgaben der Erfindung sind ferner, ein Implantat aus einem flexiblen Träger, eine Anordnung mit einem solchen Implantat sowie ein Verfahren zur Herstellung dieser Anordnung zur Verfügung zu stellen.

Diese Aufgaben werden erfindungsgemäß gelöst durch einen Träger gemäß Hauptanspruch, ein Implantat und eine Anordnung gemäß Nebenansprüchen und ein Verfahren gemäß weiterem Nebenanspruch. Weitere vorteilhafte Ausgestaltungen der Erfindung finden sich in den darauf rückbezogenen Unteransprüchen.

### Gegenstand der Erfindung

Im Rahmen der Erfindung wurde gefunden, dass ein Träger, der an einem Ende eine Mehrzahl elektrisch leitender Elemente aufweist, mit einem Abstandssensor ausgerüstet werden kann, der wenigstens einen Teil der elektrisch leitenden Elemente enthält.

Unter elektrisch leitenden Elementen werden insbesondere solche Elemente verstanden, die für die primäre Funktion eines Implantats von Bedeutung sind.

Beispielsweise sind die Elektroden eines Cochleaimplantats, die den Hörnerv elektrisch stimulieren, elektrisch leitende Elemente im Sinne dieser Erfindung.

Unter einem Abstandssensor wird im Rahmen dieser Erfindung eine Vorrichtung verstanden, die ein Signal zur Verfügung stellt, welches vom Abstand zwischen dem Element und anderen Objekten abhängt. Dieses Signal ist insbesondere beim Navigieren des Trägers in schlecht einsehbaren Räumen als Hilfsmittel verwendbar.

Der erfindungsgemäße Träger bietet den Vorteil, dass sein Abstand zu anderen Objekten besonders einfach gemessen werden kann. Insbesondere Träger, die bereits für ihre primäre Funktion elektrisch leitende Elemente an einem Ende enthalten, brauchen an diesem Ende nicht modifiziert zu werden, um sie mit einem Abstandssensor auszurüsten.

In einer vorteilhaften Ausgestaltung der Erfindung ist der Träger flexibel. Unter flexibel wird im Rahmen dieser Erfindung verstanden, dass die Form des Trägers reversibel geändert werden kann, der Träger mithin elastisch ist. Insbesondere kann der Träger sich der Geometrie eines Hohlkörpers anpassen, wenn er in diesen eingeführt wird.

Hohlkörper im Sinne dieser Erfindung sind insbesondere hohle Partien des menschlichen oder tierischen Körpers. Der Gegenstand der Erfindung erstreckt sich jedoch auch auf Hohlkörper aus anderen Gebieten der Technik, etwa Rohrleitungssysteme.

Ein erfindungsgemäßer flexibler Träger lässt sich kontrollierbarer in einen Hohlkörper einführen als flexible Träger nach dem Stand der Technik. Durch den Abstandssensor wird es insbesondere möglich, den flexiblen Träger auch in sehr beengte und nicht einsehbare Hohlkörper einzuführen, ohne dass diese Hohlkörper dabei durch Kollision mit dem Träger geschädigt werden. Insbesondere lässt sich beim Einführen des Trägers in röhrenförmige, in einer Sackgasse endende Hohlkörper, wie beispielsweise die Cochlea des menschlichen Innenohrs, der Abstand zwischen dem Ende des Trägers und dem Ende des Hohlkörpers überwachen. Im Gegensatz zum Stand der Technik ist es hierbei nicht mehr erforderlich, eine Kraft auf den Träger auszuüben, um einen mechanischen Endanschlag des Implantats im Hohlkörper zu ertasten.

In einer vorteilhaften Ausgestaltung der Erfindung ist der Träger länglich ausgestaltet. Das Verhältnis seines Durchmessers zu seiner Länge beträgt 0,1 oder weniger. Ein solcher Träger lässt sich besonderes einfach in einen Hohlkörper einführen und passt sich besonders gut der Form des Hohlkörpers an.

In einer vorteilhaften Ausgestaltung der Erfindung handelt es sich bei dem flexiblen Träger um ein Implantat. Implantate unterliegen der Anforderung, dass sie eine möglichst geringe Baugröße aufweisen müssen. Hier bietet das erfindungsgemäße Implantat den Vorteil, dass an seinem Ende keine zusätzlichen Mittel erforderlich sind, um den Abstand zwischen dem Ende des Implantats und anderen Objekten zu bestimmen. Dadurch wird der Einbauraum, den derartige Mittel einschließlich der für ihren Betrieb notwendigen Versorgungs- und Signalleitungen einnehmen, eingespart. Insbesondere ist es beim Einführen des Implantats in einen Hohlkörper auch nicht erforderlich, zusätzliche externe Geräte zur Abstandsmessung in den Hohlkörper einzuführen. Dadurch kann das Implantat auch in Hohlkörper eingeführt werden, in denen der für solche externen Geräte erforderliche zusätzliche Raum fehlt. Im Vergleich zu Implantaten nach dem Stand der Technik, die für die Abstandssensorik zusätzliche Mittel benötigen, kann das erfindungsgemäße Implantat zudem bei gleicher Baugröße im Hinblick auf seine primäre Funktion leistungsfähiger ausgestattet werden. Bei vorgegebenen Leistungsspezifikationen ist ein erfindungsgemäßes Implantat kompakter als ein Implantat nach dem Stand der Technik. Es kann somit insbesondere weniger invasiv in den menschlichen oder tierischen Körper eingeführt werden, was das Infektionsrisiko des Eingriffs verringert.

Ferner ist es auch wirtschaftlich, dass das erfindungsgemäße Implantat zum Zwecke der Abstandssensorik die an seinem Ende ohnehin vorhandenen Elektroden nutzt. Insbesondere Implantate für die Anwendung am Menschen oder Tier werden nur sehr selten, im Extremfall nur ein einziges Mal, eingeführt. Entsprechend selten wird die Abstandssensorik eingesetzt. Beim erfindungsgemäßen Implantat werden die für die Abstandssensorik verwendeten Mittel nach dem Einführen für die primäre Funktion des Implantats weitergenutzt, während solche Mittel bei Implantaten nach dem Stand der Technik nach dem Einführen brach liegen.

Der Abstandssensor enthält eine zwischen mindestens ein erstes elektrisch leitendes Element und Masse geschaltete Wechselspannungsquelle sowie ein zwischen mindestens ein zweites elektrisch leitendes Element und Masse geschaltetes Strommessgerät. Mit einer solchen Anordnung lässt sich über das erste elektrisch leitende Element der Bereich unmittelbar vor dem Implantat mit einer Wechselspannung beaufschlagen. Es wurde erkannt, dass sich über den durch das zweite elektrisch leitende Element fließenden Strom der Abstand zwischen dem Ende des Implantats und einem Hindernis bestimmen lässt.

Der Abstand wird somit rein elektrisch bestimmt, und zwar mit Hilfe problemlos verfügbarer, bewährter Standardgeräte. Die Messung ist somit einfach und kostengünstig. Zudem erfolgt sie in Echtzeit. Dies ist beim Einführen des Implantats besonders vorteilhaft, da nach der Erkennung eines Hindernisses unmittelbar vor dem Implantat noch genug Reaktionszeit verbleibt, um eine Kollision des Implantats mit dem Hindernis zu vermeiden. Zudem stehen für rein elektrische Messungen Rauschfilter in verschiedenen Varianten, insbesondere Lock-in-Verstärker, zur Verfügung. Rein elektrische Messungen lassen sich somit besonders unempfindlich gegen Rauschen ausgestalten.

Vorteilhaft ist die Wechselspannungsquelle frequenzvariabel ausgestaltet, insbesondere im Frequenzbereich zwischen 1 kHz und 1 MHz. Wie deutlich das Signal bei Annäherung des Implantats an ein Hindernis ist, hängt von der Frequenz der Wechselspannung, dem das Ende des Implantats umgebenden Material sowie vom Material des Hindernisses ab. Zudem produziert etwa ein menschlicher oder tierischer Körper selbst elektrische Impulse, die Messungen bestimmter Frequenzen stören können. Daher ist es vorteilhaft, die Frequenz durchstimmen zu können, um bei derjenigen Frequenz messen zu können, die im konkreten Anwendungsfall das beste Signal-Rausch-Verhältnis bietet. Aus der Abhängigkeit des Signals von der Frequenz lassen sich zudem im Wege der Spektroskopie möglicherweise über den Abstand zwischen Implantat und Hindernis hinaus weitere Informationen gewinnen, etwa über den Zustand des Gewebes in der Umgebung des Implantats.

Der Frequenzbereich von 1 kHz bis 1 MHz ist insoweit technisch besonders gut erschlossen, als für diesen Bereich sowohl qualitativ hochwertige Quellen als auch sehr empfindliche und rauscharme Strommessgeräte verfügbar sind. Zudem ist dieser Frequenzbereich relativ schwach mit elektromagnetischen Störungen aus der Umgebung, insbesondere aus im Labor betriebenen Funktelefonen oder PCs, verschmutzt.

Wenngleich der Frequenzbereich von 1 kHz bis 1 MHz besonders vorteilhaft ist, ist es auch denkbar, dass die Abstandsmessung in einem wesentlich weiteren Frequenzbereich von 1 mHz bis 100 MHz Spezialanwendungen findet.

Die Wechselspannungsquelle bewirkt, dass mindestens zwei elektrisch leitende Elemente auf einem gemeinsamen konstanten Potential gehalten werden. Es wurde erkannt, dass dies die Genauigkeit von Abstandsmessungen insbesondere dann steigert, wenn sich in der Umgebung um das Ende des Implantats viel elektrisch leitfähiges Material befindet, wie etwa ein Elektrolyt mit einer besonders hohen Ionenleitfähigkeit. Derartiges Material kann nämlich zu einem Spannungsabfall zwischen Wechselspannungsquelle und Strommessgerät führen. Ein solcher Spannungsabfall kann insbesondere Messungen der Impedanz, also des Verhältnisses von gemessenem Strom zu angelegter Spannung, deutlich verfälschen. Solche Verfälschungen werden vermieden, indem die Wechselspannungsquelle beispielsweise das mit ihr verbundene elektrisch leitende Element und das mit dem Strommessgerät verbundene elektrisch leitende Element auf gleichem Potential hält. Hierfür kann beispielsweise ein Potentiostat verwendet werden.

In einer weiteren vorteilhaften Ausgestaltung des Implantats enthält das Strommessgerät eine Auswerteeinheit mit Mitteln, die ein mathematisches Modell des Implantats erzeugen. Dadurch können Störeinflüsse, die das Signal-Rausch-Verhältnis der Abstandsmessung verschlechtern oder gar den Wert des gemessenen Abstands verfälschen, unmittelbar herauskorrigiert werden. Insbesondere können Störeinflüsse durch parasitäre Kapazitäten in den Zuleitungen oder Übergangswiderstände an den elektrisch leitenden Elementen minimiert werden. Zudem erleichtert ein mathematisches Modell des Implantats, etwa durch ein Widerstände und Kapazitäten enthaltendes Ersatzschaltbild, die Kalibrierung des Abstandssensors.

Vorteilhaft sind die Verbindungen zwischen dem ersten elektrisch leitenden Element und der Wechselspannungsquelle und/oder zwischen dem zweiten elektrisch leitenden Element und dem Strommessgerät lösbar ausgestaltet.

Nachdem das Implantat eingeführt worden ist, werden sowohl die Wechselspannungsquelle als auch das Strommessgerät in der Regel für den normalen Betrieb des Implantats nicht mehr benötigt. Je nach Baugröße dieser Geräte ist es daher vorteilhaft, sie nach dem Einführen des Implantats vom Implantat abkoppeln zu können. Dadurch muss etwa ein Mensch, dem ein solches Implantat eingesetzt wurde, diese Geräte nicht ständig mit sich führen. Stattdessen können die Geräte zum Einführen des nächsten Implantats an einem anderen Patienten wieder verwendet werden.

In einer besonders vorteilhaften Ausgestaltung der Erfindung weist das Implantat Mittel zur Änderung der Form, insbesondere der Krümmung, des Trägers auf. Dadurch kann das Implantat einem Hindernis, das durch den Abstandssensor erkannt wird, ausweichen. Dies ist insbesondere beim Einführen des Implantats in einen stark beengten und nicht einsehbaren Hohlkörper vorteilhaft, der zudem bei Kollision mit dem Implantat verletzt werden könnte. Ein derartiger Hohlkörper ist beispielsweise die Cochlea des menschlichen Innenohrs.

Die Mittel zur Änderung der Form sind vorteilhaft hydraulische oder pneumatische Mittel. Derartige Mittel, wie sie etwa aus den Offenlegungsschriften DE 198 43 739 A1, DE 198 24 622 A1 und DE 103 16 959 A1 bekannt sind, zeichnen sich durch eine besonders geringe Baugröße aus. Zudem kann ein Teil der Komponenten, die für ihren Betrieb erforderlich sind, außerhalb des Implantats angeordnet sein. Einige dieser Komponenten können auch außerhalb des Hohlkörpers verbleiben, in den das Implantat eingeführt wird, was den Eingriff drastisch vereinfacht. Das hydraulische beziehungsweise pneumatische Betriebsmittel kann außerdem nach dem Einführen des Implantats aus diesem entfernt werden, wodurch das Implantat in seiner endgültigen Position besonders wenig Raum beansprucht.

Vorteilhaft ist eine Steuereinheit für die Mittel zur Änderung der Form vorgesehen. Dadurch kann gezielt Einfluss auf die Form des Implantats genommen werden. Die Steuereinheit lässt sich insbesondere außerhalb des Hohlkörpers anordnen, in den das Implantat eingeführt wird, so dass nur das Implantat selbst in den Hohlkörper gelangt. Handelt es sich bei dem Hohlkörper um eine Partie des menschlichen oder tierischen Körpers, wird der Eingriff dadurch minimal invasiv, was das Infektionsrisiko verringert.

Die Verbindung zwischen der Steuereinheit und den Mitteln zur Änderung der Form ist vorteilhaft lösbar ausgestaltet. Das Steuergerät wird in aller Regel nur einmal zum Einführen des Implantats benötigt, nicht jedoch für den weiteren Betrieb des Implantats. Sind die Mittel lösbar, lässt sich das Steuergerät nach dem Einführen des Implantats vom Implantat trennen. Dadurch muss insbesondere ein Mensch, der ein medizinisches Implantat trägt, das Steuergerät nicht ständig bei sich führen. Zudem kann das Steuergerät für das Einführen des nächsten Implantats wieder verwendet werden.

In einer besonders vorteilhaften Ausgestaltung der Erfindung ist ein Kommunikationskanal zwischen Steuereinheit und Strommessgerät angeordnet. Es wurde erkannt, dass sich dadurch die Vermeidung von Kollisionen des Implantats mit Hindernissen automatisieren lässt. Der Operateur braucht das Implantat nur noch in den Hohlkörper hinein zu schieben; es biegt sich selbsttätig um eventuelle Hindernisse herum. Der Eingriff wird dadurch wesentlich weniger fehleranfällig, da der Operateur sich nur noch auf den Vortrieb konzentrieren muss. Somit wird das Risiko von Beschädigungen beziehungsweise Verletzungen des Hohlkörpers beim Einführen des Implantats vermindert.

In einer besonders vorteilhaften Ausgestaltung der Erfindung ist das Implantat ein Cochleaimplantat. Es wurde erkannt, dass die Vorteile der erfindungsgemäßen Implantate bei Cochleaimplantaten in besonderem Maße zum Tragen kommen: Die Cochlea des menschlichen Innenohrs ist beengt, nicht einsehbar, endet in einer Sackgasse und ist zudem empfindlich gegenüber Kollisionen mit einem Implantat. Zudem muss ein Loch in den Schädel gebohrt werden, um überhaupt einen Zugang zur Cochlea zwecks Einführung des Implantats zu bekommen. Dadurch ist es nur sehr schwer möglich, zusätzliche Technik einzuführen, um die Einführung des Implantats zu überwachen. Ein erfindungsgemäßes Cochleaimplantat nutzt die bereits am Implantat vorhandene Technik für die Abstandssensorik. Lediglich zur Änderung der Form des Trägers sind gegebenenfalls noch zusätzliche Mittel erforderlich; hierfür werden Lösungen mit minimalem Raumbedarf zur Verfügung gestellt. Der größte Teil der Geräte, mit denen das Einführen des Implantats überwacht wird, kann außerhalb des Körpers des Patienten verbleiben. Somit ist im Gegensatz zum Stand der Technik keine größere oder zusätzliche Öffnung im Schädel erforderlich, um das Einführen des Implantats überwachen zu können. Die erfindungsgemäße Überwachung des Einführens verringert das Verletzungsrisiko deutlich, insbesondere wenn sich das Ende des Implantats dem Ende der Cochlea nähert. Dadurch ist es möglich, das Ende des Implantats zielgenau möglichst nahe an das Ende der Cochlea heranzuführen. Der durch das Implantat bewirkte Höreindruck wird so qualitativ verbessert. Zudem vereinfacht die Überwachung den Eingriff, so dass dieser in wesentlich kürzerer Zeit und somit auch mit wesentlich kürzerer Narkose durchgeführt werden kann.

Im Rahmen der Erfindung wurde gefunden, dass eine Anordnung aus einem Implantat, einem dieses umgebenden Hohlkörper, wobei hohle Partien des menschlichen oder tierischen Körpers ausgenomen sind, sowie einem Elektrolyten dadurch verbessert werden kann, dass als Implantat ein erfindungsgemäßes Implantat nach einem der Ansprüche 1 bis 13 verwendet wird.

Unter Elektrolyt ist jeder Stoff zu verstehen, der zumindest teilweise in Form von Ionen vorliegt. Beispiele für Elektrolyte sind Festkörperelektrolyte, gelöste Substanzen und Oberflächenbeschichtungen. Der Hohlkörper kann also beispielsweise aus einem Metall mit einer Schicht bestehen, die mit Hilfe des Implantats zu charakterisieren ist.

Da sich das Implantat zielgenau bis an die Stelle des Hohlkörpers einführen lässt, an der es seine Wirkung optimal entfalten kann, wirkt das Implantat in einer derartigen Anordnung besonders effizient. Zudem weist der Hohlkörper in einer solchen Anordnung mit einer geringeren Wahrscheinlichkeit Schäden auf, die in Anordnungen nach dem Stand der Technik beim Einführen des Implantats entstehen. Die Anordnung ist wesentlich einfacher, schneller und kostengünstiger herzustellen als vergleichbare Anordnungen nach dem Stand der Technik.

In einer besonders vorteilhaften Ausgestaltung der Erfindung ist der Elektrolyt eine menschliche oder tierische Körperflüssigkeit, insbesondere Perilymphe. Somit kann das Implantat vorteilhaft etwa ein Katheter oder ein chirurgisches Instrument und der Hohlkörper ein beengter Bereich des menschlichen oder tierischen Körpers sein. Das Implantat lässt sich im Hohlkörper navigieren, ohne dass dieser mit Röntgenstrahlen, Kontrastmitteln oder anderen Fremdsubstanzen belastet werden muss. Zudem benötigt die Anordnung keinen künstlichen Elektrolyten, der insbesondere bei einer Leckage des Hohlkörpers Gesundheitsschäden hervorrufen könnte.

Die Verwendung von Perilymphe bietet den besonderen Vorteil, dass als Hohlkörper die Cochlea des menschlichen Innenohrs gewählt werden kann. Diese ist regelmäßig mit Perilymphe gefüllt. In dieser Ausführungsform kann das Implantat insbesondere genau dosiert bis zum Ende der Cochlea eingeführt werden, ohne beim Endanschlag eine mechanische Kraft auf die Cochlea auszuüben.

Im Rahmen der Erfindung wird ein Verfahren zur Herstellung der erfindungsgemäßen Anordnung, welche nach einem der Ansprüche 14 oder 15 ausgebildet ist, zur Verfügung gestellt. Dabei wird das Implantat in einem ersten Schritt in den Hohlkörper hineinbewegt. In einem zweiten Schritt wird es in einer Weise gekrümmt, dass sein in Bewegungsrichtung vorderes Ende die Wandung des Hohlkörpers nicht berührt. Die beiden Schritte werden abwechselnd durchgeführt, bis die Anordnung fertig ist. Das Verfahren hat den Vorteil, dass es einfacher und schneller ist als Verfahren nach dem Stand der Technik, bei denen das Implantat mit mechanischer Andruckkraft um Biegungen im Hohlkörper gezwungen wird. Dadurch wird auch das Risiko einer Beschädigung des Hohlkörpers minimiert.

Vorteilhaft wird die Berührung zwischen dem Ende des Implantats und der Wandung des Hohlkörpers vermieden, indem der Abstand zwischen dem Ende des Implantats und der Wandung des Hohlkörpers gemessen wird. Dieser Abstand ist eine leicht interpretierbare Größe, die dem Operateur das Risiko einer Berührung besonders veranschaulicht. Daraufhin kann dieser die notwendigen Maßnahmen ergreifen. Beispielsweise kann er das Implantat um ein Hindernis herumführen.

Vorteilhaft wird ein abnehmender Abstand zumindest teilweise durch eine Änderung der Form, insbesondere der Krümmung, des Implantats kompensiert. Dadurch wird es möglich, das Implantat um ein Hindernis herumzuführen. Insbesondere kann das Implantat so in einen stark verwinkelten oder gewundenen Hohlkörper, wie etwa die Cochlea des menschlichen Innenohrs, eingeführt werden.

In einer besonders vorteilhaften Ausgestaltung der Erfindung wird der Abstand bestimmt, indem an mindestens einem Einspeiseort eine Wechselspannung gegen Masse angelegt und der Strom zwischen mindestens einem Messort und Masse gemessen wird. Es wurde erkannt, dass sich aus der Abhängigkeit des gemessenen Stroms von der angelegten Spannung (Impedanz) der Abstand zwischen dem Ende des Implantats und der Wandung des Hohlkörpers bestimmen lässt.

Hierbei ist der in der Anordnung enthaltene Elektrolyt notwendig, damit die am Einspeiseort angelegte Spannung zu einem Stromfluss durch den Messort führen kann.

Die für den Zweck der Abstandsmessung optimale Frequenz der angelegten Wechselspannung hängt von der Geometrie und den Materialien der Anordnung sowie insbesondere von der Art des Elektrolyten ab. Sie kann für eine gegebene Anordnung durch den Fachmann in einer zumutbaren Anzahl Versuche bestimmt werden.

Der Stromfluss durch den Messort hängt von der am Einspeiseort angelegten Spannung ab. Diese Beziehung ist frequenzabhängig und wird als Wechselstromwiderstand (Impedanz) bezeichnet. Es wurde erkannt, dass sich diese Impedanz in charakteristischer Weise ändert, wenn sich der Abstand zwischen dem Ende des Implantats und der Wandung des Hohlkörpers ändert. Daher kann durch Bestimmung der Impedanz der Abstand zwischen dem Ende des Implantats und der Wandung des Hohlkörpers bestimmt werden. Um den Abstand absolut zu bestimmen, muss für eine gegebene Anordnung in der Regel zunächst eine Eichung durchgeführt werden.

Der Vorteil dieser Messmethode liegt darin, dass sie am Ort der Messung keine komplizierten technischen Einrichtungen erfordert. Dadurch kann die Messung auch in räumlich sehr beengten Anordnungen durchgeführt werden. Zudem funktioniert sie auf elektrischer Basis und damit in Echtzeit. Es ist also mit dieser Messmethode insbesondere möglich, Implantate in räumlich beengten und nicht direkt einsehbaren Anordnungen, wie etwa Rohrsystemen, zu navigieren.

Die Frequenz der Wechselspannung kann insbesondere zwischen 1 kHz und 1 MHz betragen. In diesem Frequenzbereich ist für gängige Elektrolyten ein gutes Signal zu erwarten, während nur geringe Störeinflüsse durch Fremdeinstrahlung zu befürchten sind. Im Ergebnis ist das Signal-Rausch-Verhältnis vorteilhaft.

Wenngleich der Frequenzbereich von 1 kHz bis 1 MHz besonders vorteilhaft ist, ist es auch denkbar, dass die Abstandsmessung in einem wesentlich weiteren Frequenzbereich von 1 mHz bis 100 MHz Spezialanwendungen findet.

Vorteilhaft hat die Wechselspannung eine Amplitude zwischen 2 mV und 10 mV. Derart kleine Spannungen sind biokompatibel, so dass das Verfahren zum Einsatz am und innerhalb des menschlichen oder tierischen Körpers geeignet ist.

Vorteilhaft wird vor der eigentlichen Messung die Anordnung durch ein Widerstände und Kapazitäten enthaltendes Ersatzschaltbild modelliert, wobei die Parameter der Schaltung solange variiert werden, bis das Impedanzverhalten des Modells und der Anordnung möglichst gut übereinstimmen. Dadurch können Einflusse der einzelnen Komponenten in der Anordnung, wie etwa Zuleitungen, Schichtkapazitäten oder Durchtrittswiderstände, auf das Messergebnis bestimmt werden. Es können auch Einflüsse systemspezifischer Parameter, wie etwa der Porosität von Oberflächen, der Zusammensetzung des Elektrolyten, des Materials der elektrisch leitenden Elemente sowie der Größe und Form der elektrisch leitenden Elemente auf das Messergebnis bestimmt werden. Dadurch können diese Einflüsse aus den erhaltenen Messergebnissen herauskorrigiert werden, was die Genauigkeit der Messung steigert. Insbesondere wird durch ein geeignetes Modell die quantitative Kalibrierung der Anordnung wesentlich erleichtert, so dass den erhaltenen Messergebnissen ein absoluter Abstand zugeordnet werden kann. Durch die Auswahl geeigneter Betriebsparameter kann dann eine einfache Ausleseelektronik für die Messung erstellt werden.

Vorteilhaft werden mindestens ein Einspeiseort und mindestens ein Messort auf einem gemeinsamen konstanten Potential gehalten. Dies kann beispielsweise durch einen Potentiostaten bewirkt werden. Auf diese Weise wird ein durch einen Stromfluss zwischen Einspeiseort und Messort bewirkter Spannungsabfall ausgeglichen. Dadurch wird eine Verfälschung des Messergebnisses vermieden, insbesondere wenn der Elektrolyt eine sehr hohe Ionenleitfähigkeit aufweist.

Vorteilhaft wird die Messung mit mindestens zwei verschiedenen Frequenzen der angelegten Wechselspannung durchgeführt. Hierzu kann etwa mit einem Frequenzgang-Analysator die Frequenz der angelegten Wechselspannung durchgestimmt und die Impedanz frequenzabhängig aufgenommen werden. Man spricht in diesem Fall von Impedanz-Spektroskopie. Auf diese Weise entfällt die Notwendigkeit, zunächst manuell die optimale Frequenz für die gegebene Messanordnung bestimmen zu müssen. Zudem wird die Messung deutlich weniger fehleranfällig, weil der Abstand auch über charakteristische Merkmale des Frequenzgangs der Impedanz bestimmt werden kann. Dieser ist vergleichsweise unempfindlich gegenüber Messfehlern bei einzelnen Messwerten.

In einer weiteren Ausgestaltung der Erfindung wird die Wechselspannung an mehreren Einspeiseorten angelegt und/oder der Strom zwischen mehreren Messorten und Masse gemessen. Dadurch kann beispielsweise die Messung entlang einer Linie durchgeführt werden, wodurch sie wesentlich weniger fehleranfällig wird als eine Messung an lediglich einem Punkt. Durch eine zweidimensionale Anordnung der Einspeiseorte und/oder Messorte können auch flächige Abstandsmessungen durchgeführt werden. Dies ist besonders vorteilhaft, um Kollisionen zwischen dem ausgedehnten Ende des Implantats und der ebenfalls ausgedehnten Wandung des Hohlkörpers zu vermeiden. Es können auch Anordnungen realisiert werden, bei denen nicht alle Mess- und/oder Einspeiseorte den gleichen Abstand gegenüber einer Bezugsfläche haben. Hierdurch kann unterschiedlich empfindlich auf Annäherungen verschiedener Stellen von Ende des Implantats und Wandung des Hohlkörpers reagiert werden. Dies ist insbesondere dann vorteilhaft, wenn ein Implantat in einem beengten Hohlkörper navigiert wird und in verschiedenen Freiheitsgraden verschieden schnell bewegt werden kann.

Die Einspeiseorte und/oder die Messorte sind vorzugsweise jeweils Elektroden am Ende des Implantats und/oder an der Wandung des Hohlkörpers. Dadurch können auch Abstandsmessungen durchgeführt werden, wenn das Ende des Implantats oder die Wandung des Hohlkörpers elektrisch isolierend ist. Elektroden stellen zudem definierte Wechselwirkungszentren dar und erhöhen die Genauigkeit der Messung. Elektroden können vorteilhaft bezüglich Querschnittsfläche, Oberflächenform und Material auf die jeweilige Anordnung optimiert werden.

In einer besonders vorteilhaften Ausgestaltung der Erfindung befinden sich sämtliche Einspeise- und Messorte auf dem Implantat. Dadurch muss die Wandung des Hohlkörpers nicht elektrisch kontaktiert werden, was in beengten Raumverhältnissen nicht immer möglich ist. Die Wandung des Hohlkörpers muss auch nicht elektrisch leitend oder gar geerdet sein, was den Anwendungsbereich des Messverfahrens deutlich erweitert.

Es wurde erkannt, dass sich die an einem Cochleaimplantat für das menschliche Innenohr ohnehin vorhandenen Elektroden als Einspeise- und/oder Messorte verwenden lassen, um mit dem erfindungsgemäßen Verfahren den Abstand zwischen dem Ende des Implantats und der Wandung der Cochlea zu bestimmen. Der Vorteil liegt darin, dass am Implantat selbst für die Abstandssensorik keinerlei weitere Vorrichtungen angebracht werden müssen, für die in der sehr beengten Cochlea kein Raum ist. Stattdessen befindet sich sämtliche für die Abstandssensorik erforderliche zusätzliche Technik außerhalb des Patienten.

Die Erfindung ist nicht darauf beschränkt, lediglich den Abstand zwischen dem Ende des Implantats und der Wandung der Cochlea zu bestimmen. Sind weitere Bereiche des Implantats mit Elektroden ausgestattet, kann auch der Abstand zwischen diesen Bereichen und der Wandung der Cochlea bestimmt werden.

### Spezieller Beschreibungsteil

Nachfolgend wird der Gegenstand der Erfindung anhand von Figuren näher erläutert, ohne dass der Gegenstand der Erfindung dadurch beschränkt wird. Es zeigen:
- Figur 1:: Prinzipielle Darstellung einer Anordnung, in der der Abstand zwischen Implantat und Wandung des Hohlkörpers bestimmt wird.
- Figur 2:: Nyquist-Plots für eine Anordnung mit zwei Kupferelektroden von 100 µm Durchmesser in 0,8 mm Abstand in Perilymphe.
- Figur 3:: Bode-Plot für die in Figur 2 verwendete Anordnung.

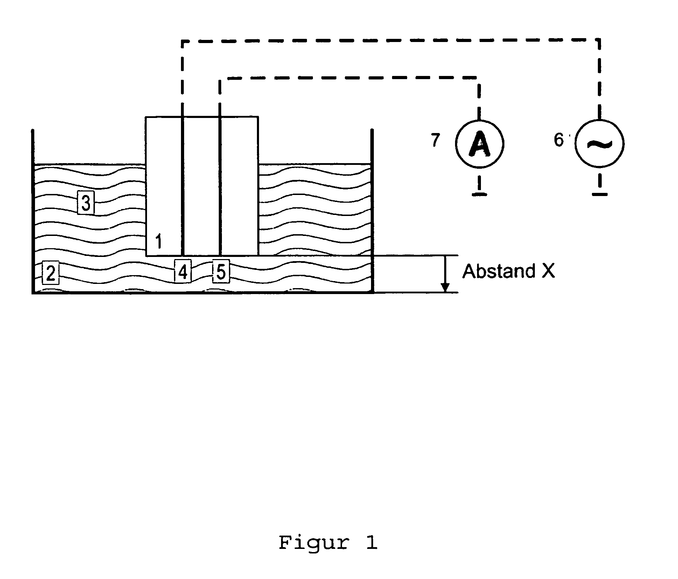

Figur 1 zeigt den prinzipiellen Aufbau einer Anordnung, in der der Abstand zwischen dem Ende des Implantats und der Wandung des Hohlkörpers gemessen wird. In Figur 1 ist die Länge des Implantats aus Gründen der Platzersparnis stark verkürzt eingezeichnet. In diesem Beispiel ist der Elektrodenhalter 1 das Implantat; der mit dem Elektrolyten 3 gefüllte Behälter 2 ist der Hohlkörper. Der Einspeiseort 4 und der Messort 5 bestehen aus jeweils einer auf der Unterseite des Elektrodenhalters aufgebrachten Elektrode. Sie befinden sich somit beide auf dem Implantat. Der Einspeiseort wird über eine Wechselspannungsquelle 6 mit einer Wechselspannung gegen Masse beaufschlagt. Der Strom zwischen dem Messort und Masse wird mit einem Strommessgerät 7 gemessen. Jede Änderung des Abstands X zwischen der Unterseite des Elektrodenhalters, die das Ende des Implantats repräsentiert, und dem Boden des Behälters führt zu einer Impedanzänderung, an Hand derer der Abstand bestimmt werden kann.

In einem Ausführungsbeispiel wurde mit diesem Aufbau bei unterschiedlichen Abständen X die Impedanz ermittelt. Dabei wurden als Elektroden zwei in einem Abstand von 0,8 mm angeordnete Kupferelektroden mit je 100 µm Durchmesser verwendet. Es wurde ein Elektrolyt verwendet, der in seiner Zusammensetzung der in der menschlichen Cochlea vorhandenen Perilymphe entspricht. Die beiden Elektroden wurden durch einen in Figur 1 nicht eingezeichneten Potentiostaten auf gleichem Potential gehalten.

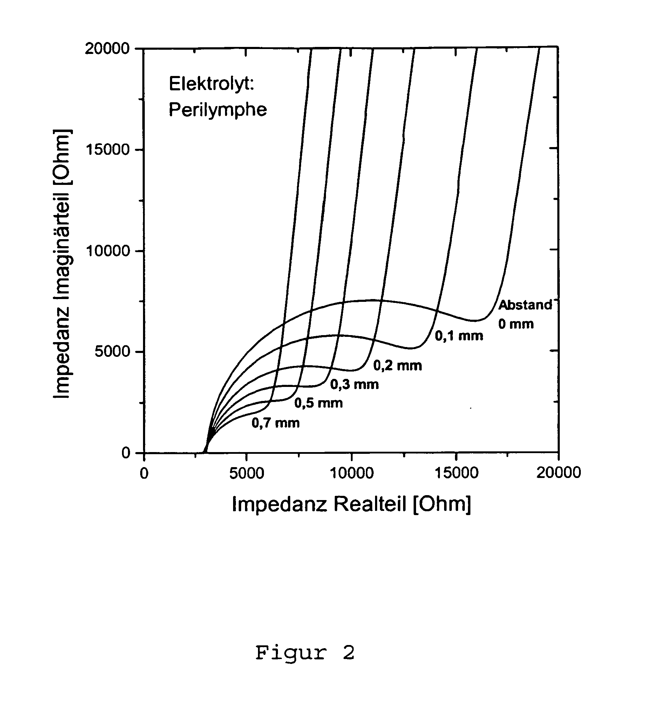

Für Abstände X zwischen 0 und 0,7 mm wurde jeweils die Frequenz der Wechselspannung von 1000 bis 100000 Hz mit einem in Figur 1 nicht eingezeichneten Frequenzganganalysator durchgestimmt und die Impedanz der Anordnung gemessen. In Figur 2 sind Imaginär- und Realteil der gemessenen Impedanzen jeweils gegeneinander aufgetragen. Die Abhängigkeit des Imaginärteils vom Realteil ist für die verschiedenen Abstände X qualitativ ähnlich, quantitativ jedoch höchst unterschiedlich. Somit lässt sich eine vergleichsweise einfache Ausleseelektronik realisieren und so kalibrieren, dass der Abstand zwischen Ende des Implantats und Wandung des Hohlkörpers auch absolut gemessen werden kann. Eine solche Ausleseelektronik könnte beispielsweise die Position auswerten, an der im rechten Teil der Kurven jeweils ein sehr steiler Anstieg einsetzt.

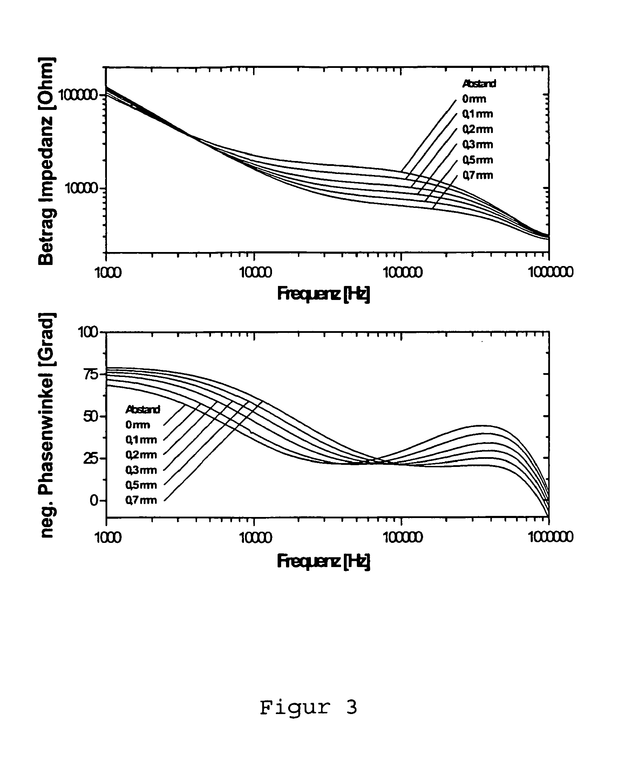

Die gleichen Messergebnisse sind in Figur 3 in Form eines Bode-Plots dargestellt. In einem solchen Plot sind jeweils der Betrag der Impedanz und der negative Phasenwinkel als Funktionen der Frequenz der Wechselspannung dargestellt. Die Kurven zeigen für die verschiedenen Abstände X wiederum qualitativ ähnliche, quantitativ jedoch unterschiedliche Verläufe. Es fällt auf, dass die Unterschiede je nach Frequenz sehr unterschiedlich stark ausgeprägt sind und sich die Kurven für verschiedene Abstände sogar schneiden. Das bedeutet, dass die qualitative Abhängigkeit des Betrags der Impedanz beziehungsweise des negativen Phasenwinkels vom Abstand sich bei bestimmten Frequenzen umkehrt. Die Bode-Plots sind also ein gutes Hilfsmittel, um für eine Anordnung die optimale Messfrequenz zu bestimmen. Die für die Auswertung herangezogene Messgröße (Betrag der Impedanz oder negativer Phasenwinkel) sollte bei der Messfrequenz möglichst stark vom Abstand abhängen. Auf diese Weise können Fehlmessungen vermieden werden. Es wurde zudem erkannt, dass weniger Fehlmessungen bei einer Messfrequenz auftreten, die weit von einer Frequenz entfernt ist, bei der sich die Abstandsabhängigkeit qualitativ umkehrt.

Die Erfindung ist auf das vorher beschriebene Verfahren zur Herstellung der Anordnung nicht beschränkt. Vielmehr wird im Rahmen der Erfindung ein generelles Verfahren zur Messung des Abstands zweier Körper bereitgestellt. Es wird vorausgesetzt, dass einer der Körper mindestens zwei Elektroden umfasst und der Raum zwischen den beiden Körpern wenigstens teilweise mit einem Elektrolyten gefüllt ist. Eine Änderung des Abstands zwischen den beiden Körpern führt zu einer Änderung der Impedanz des Systems. Die Analyse der Impedanz ermöglicht eine Messung des Abstands.

## Patentansprüche

1. Implantat (1), umfassend einen Träger mit einer Mehrzahl elektrisch leitender Elemente (4,5) an einem Ende, wobei der Träger einen Abstandssensor aufweist, der wenigstens einen Teil der elektrisch leitenden Elemente (4,5) enthält,
**gekennzeichnet durch**
eine zwischen mindestens ein erstes elektrisch leitendes Element (4) und Masse geschaltete Wechselspannungsquelle (6) sowie **durch** ein zwischen mindestens ein zweites elektrisch leitendes Element (5) und Masse geschaltetes Strommessgerät (7), wobei die Wechselspannungsquelle (6) bewirkt, dass mindestens zwei elektrisch leitende Elemente (4,5) auf einem gemeinsamen konstanten Potential gehalten werden.

2. Implantat (1) nach Anspruch 1, **dadurch gekennzeichnet, dass** der Träger flexibel ausgebildet ist.

3. Implantat (1) nach einem der Ansprüche 1 bis 2, **gekennzeichnet durch** einen Träger von länglicher Form mit einem Verhältnis von Durchmesser zu Länge von 0,1 oder weniger.

4. Implantat (1) nach einem der Ansprüche 1 bis 3, **gekennzeichnet durch** eine frequenzvariable Wechselspannungsquelle, insbesondere im Frequenzbereich zwischen 1 kHz und 1 MHz.

5. Implantat (1) nach einem der Ansprüche 1 bis 4, **gekennzeichnet durch** ein Strommessgerät (7), enthaltend eine Auswerteeinheit mit Mitteln, die ein mathematisches Modell des Implantats (1) erzeugen.

6. Implantat (1) nach einem der Ansprüche 1 bis 5, **gekennzeichnet durch** lösbare Verbindungen zwischen dem ersten elektrisch leitenden Element (4) und der Wechselspannungsquelle (6) und/oder zwischen dem zweiten elektrisch leitenden Element (5) und dem Strommessgerät (7).

7. Implantat (1) nach einem der Ansprüche 1 bis 6, **gekennzeichnet durch** Mittel zur Änderung der Form, insbesondere der Krümmung, des Trägers.

8. Implantat (1) nach Anspruch 7, **gekennzeichnet durch** hydraulische Mittel zur Änderung der Form des Trägers.

9. Implantat (1) nach einem der Ansprüche 7 bis 8, **gekennzeichnet durch** pneumatische Mittel zur Änderung der Form des Trägers.

10. Implantat (1) nach einem der Ansprüche 7 bis 9, **gekennzeichnet durch** eine Steuereinheit für die Mittel zur Änderung der Form des Trägers.

11. Implantat (1) nach Anspruch 10, **gekennzeichnet durch** eine lösbare Verbindung zwischen der Steuereinheit und den Mitteln zur Änderung der Form des Trägers.

12. Implantat (1) nach einem der Ansprüche 10 bis 11, **gekennzeichnet durch** einen Kommunikationskanal zwischen Steuereinheit und Strommessgerät (7).

13. Cochleaimplantat als Implantat (1) nach einem der Ansprüche 1 bis 12.

14. Anordnung aus einem Implantat (1), einem dieses umgebenden Hohlkörper (2), wobei hohle Partien des menschlichen oder tierischen Körpers ausgenommen sind, sowie einem Elektrolyten (3), **gekennzeichnet durch** ein Implantat (1) nach einem der Ansprüche 1 bis 13.

15. Anordnung nach Anspruch 14, **gekennzeichnet durch** eine Körperflüssigkeit, insbesondere Perilymphe, als Elektrolyten (3).

16. Verfahren zur Herstellung einer Anordnung nach einem der Ansprüche 14 bis 15 mit den Schritten:
- das Implantat (1) wird in den Hohlkörper (2) hineinbewegt;
- das Implantat (1) wird in einer Weise in seiner Form geändert, insbesondere gekrümmt, dass sein in Bewegungsrichtung vorderes Ende die Wandung des Hohlkörpers (2) nicht berührt.

17. Verfahren nach Anspruch 16, **dadurch gekennzeichnet, dass** der Abstand zwischen dem Ende des Implantats (1) und der Wandung des Hohlkörpers (2) gemessen wird.

18. Verfahren nach einem der Ansprüche 16 bis 17, **dadurch gekennzeichnet, dass** ein abnehmender Abstand zumindest teilweise durch eine Formänderung des Implantats (1) kompensiert wird.

19. Verfahren nach einem der Ansprüche 16 bis 18, **dadurch gekennzeichnet, dass** der Abstand nach einem Verfahren mit den folgenden Schritten gemessen wird:
- an mindestens einem Einspeiseort (4) wird eine Wechselspannung gegen Masse angelegt;
- es wird der Strom zwischen mindestens einem Messort (5) und Masse gemessen;
- aus der Abhängigkeit des gemessenen Stroms von der angelegten Spannung wird der Abstand bestimmt.

20. Verfahren nach Anspruch 19, **gekennzeichnet durch** eine Wechselspannung mit einer Frequenz zwischen 1 kHz und 1 MHz.

21. Verfahren nach einem der Ansprüche 19 bis 20, **gekennzeichnet durch** eine Wechselspannung mit einer Amplitude zwischen 2 mV und 10 mV.

22. Verfahren nach einem der Ansprüche 19 bis 21, **dadurch gekennzeichnet, dass** die Anordnung aus Implantat (1) und Hohlkörper (2) vor der eigentlichen Messung auf der Basis eines Ersatzschaltbilds modelliert wird.

23. Verfahren nach einem der Ansprüche 19 bis 22, **dadurch gekennzeichnet, dass** mindestens ein Einspeiseort (4) und mindestens ein Messort (5) auf einem gemeinsamen konstanten Potential gehalten werden.

24. Verfahren nach einem der Ansprüche 19 bis 23, **dadurch gekennzeichnet, dass** die Messung mit mindestens zwei verschiedenen Frequenzen der angelegten Wechselspannung durchgeführt wird.

25. Verfahren nach einem der Ansprüche 19 bis 24, **gekennzeichnet durch** mehrere Einspeiseorte (4).

26. Verfahren nach einem der Ansprüche 19 bis 25, **gekennzeichnet durch** mehrere Messorte (5).

27. Verfahren nach einem der Ansprüche 19 bis 26, **gekennzeichnet durch** Elektroden als Einspeise- (4) und/oder Messorte (5).

28. Verfahren nach einem der Ansprüche 19 bis 27, **dadurch gekennzeichnet, dass** sich sämtliche Einspeise- (4) und Messorte (5) auf dem Implantat (1) befinden.

29. Verfahren nach Anspruch einem der Ansprüche 19 bis 28, **gekennzeichnet durch** Stimulationselektroden eines Cochleaimplantats als Einspeise- (4) und/oder Messorte (5).

## Claims

1. Implant (1), comprising a support with a multiplicity of electrically conducting elements (4,5) at one end, in which the support has a distance sensor that contains at least a part of the electrically conducting elements (4,5), **characterised in that** at least one alternating current source (6) is connected between at least a first electrically conducting element (4) and earth, as well as by a current measuring appliance (7) connected between at least a second electrically conducting element (5) and earth, in which the alternating current source (6) has the effect that at least two electrically conducting elements (4, 5) are maintained at a common constant potential.

2. Implant (1) in accordance with Claim 1, **characterised in that** the support is designed to be flexible.

3. Implant (1) in accordance with one of Claims 1 to 2, **characterised by** a support of oblong shape with a ratio of diameter to length of 0.1 or less.

4. Implant (1) in accordance with one of Claims 1 to 3, **characterised by** a variable frequency alternating current source, in particular in the frequency range between 1 kHz and 1 MHz.

5. Implant (1) in accordance with one of Claims 1 to 4, **characterised by** a current measuring device (7) containing a plotting unit with means, which produce a mathematical model of the implant (1).

6. Implant (1) in accordance with one of Claims 1 to 5, **characterised by** detachable connections between the first electrically conducting element (4) and the alternating current source (6) and/or between the second electrically conducting element (5) and the current measuring appliance (7).

7. Implant (1) in accordance with one of Claims 1 to 6, **characterised by** means for changing the shape of the support, in particular that of the curvature.

8. Implant (1) in accordance with Claim 7, **characterised by** hydraulic means for changing the shape of the support.

9. Implant (1) in accordance with one of Claims 7 to 8, **characterised by** pneumatic means for changing the shape of the support.

10. Implant (1) in accordance with one of Claims 7 to 9, **characterised by** a detachable connection between the control unit and the means for changing the shape of the support.

11. Implant (1) in accordance with Claim 10, **characterised by** a detachable connection between the control unit and the means for changing the shape of the support.

12. Implant (1) in accordance with one of Claims 10 to 11, **characterised by** a communication channel between the control unit and current measuring appliance (7).

13. Cochlea implant as an implant (1) in accordance with one of the Claims 1 to 12.

14. Arrangement of an implant (1), a hollow body surrounding this, in which hollow parts of the human or animal body are excluded, and an electrolyte (3), **characterised by** an implant (1) in accordance with one of the Claims 1 to 13.

15. Arrangement in accordance with Claim 14, **characterised by** a body fluid, in particular perilymph, as an electrolyte (3).

16. Process for producing an arrangement in accordance with one of the Claims 14 to 15 with the following stages:
- the implant (1) is moved into the hollow body (2);
- the implant (1) has its shaped changed and is bent in particular in a manner that its front end facing the direction of movement does not touch the walls of the hollow body (2).

17. Process in accordance with Claim 16, **characterised in that** the distance between the end of the implant (1) and the walls of the hollow body (2) is measured.

18. Process in accordance with one of Claims 16 to 17, **characterised in that** a decreasing distance is at least partially compensated by a change of shape in the implant (1).

19. Process in accordance with one of Claims 16 to 18, **characterised in that** the distance is measured using a process with the following stages:
- an alternating current is applied to the earth at least one power supply site (4);
- he current is measured between at least one measuring site (5) and the earth;
- the distance is determined from the dependency of the measured current on the current applied.

20. Process in accordance with Claim 19, **characterised by** an alternating current with a frequency of between 1 kHz and 1 MHz.

21. Process in accordance with one of Claims 19 to 20, **characterised by** an alternating current with an amplitude of between 2 mV and 10 mV.

22. Process in accordance with one of Claims 19 to 21, **characterised in that** the arrangement of the implant (1) and hollow body (2) is modelled prior to the actual measurement on the basis of an equivalent circuit diagram.

23. Process in accordance with one of Claims 19 to 22, **characterised in that** at least one power supply site (4) and at least one measuring site (5) are maintained at a common constant potential.

24. Process in accordance with one of Claims 19 to 23, **characterised in that** the measurement is carried out using at least two different frequencies for the alternating current supplied.

25. Process in accordance with one of Claims 19 to 24, **characterised by** several power supply sites (4).

26. Process in accordance with one of Claims 19 to 25, **characterised by** several measuring sites (5).

27. Process in accordance with one of Claims 19 to 26, **characterised by** electrodes as power supply (4) and/or measuring sites (5).

28. Process in accordance with one of Claims 19 to 27, **characterised in that** all the power supply (4) and measuring sites (5) are situated on the implant (1).

29. Process in accordance with one of Claims 19 to 28, **characterised by** stimulation electrodes of a cochlea implant as being power supply (4) and measuring sites (5).

## Revendications

1. Implant (1), comportant un support avec une pluralité d'éléments électroconducteurs (4, 5) à une extrémité, ledit support comportant un capteur de distance qui contient au moins une partie des éléments électroconducteurs (4, 5),
**caractérisé par** une source de tension alternative (6), montée entre au moins un premier élément électroconducteur (4) et la masse, ainsi que par un appareil de mesure du courant (7), monté entre au moins un deuxième élément électroconducteur (5) et la masse, ladite source de tension alternative (6) faisant en sorte qu'au moins deux éléments électroconducteurs (4, 5) soient maintenus à un potentiel constant commun.

2. Implant (1) selon la revendication 1, **caractérisé en ce que** le support est flexible.

3. Implant (1) selon l'une des revendications 1 à 2, **caractérisé par** un support de forme allongée, dont le rapport entre le diamètre et la longueur est égal à 0,1 ou moins.

4. Implant (1) selon l'une quelconque des revendications 1 à 3, **caractérisé par** une source de tension alternative à fréquence variable, en particulier dans la plage de fréquences entre 1 kHz et 1 MHz.

5. Implant (1) selon l'une quelconque des revendications 1 à 4, **caractérisé par** un appareil de mesure du courant (7) comportant une unité d'analyse avec des moyens générant un modèle mathématique de l'implant (1).

6. Implant (1) selon l'une quelconque des revendications 1 à 5, **caractérisé par** des liaisons amovibles entre le premier élément électroconducteur (4) et la source de tension alternative (6) et/ou entre le deuxième élément électroconducteur (5) et l'appareil de mesure du courant (7).

7. Implant (1) selon l'une quelconque des revendications 1 à 6, **caractérisé par** des moyens destinés à modifier la forme, en particulier la courbure, du support.

8. Implant (1) selon la revendication 7, **caractérisé par** des moyens hydrauliques destinés à modifier la forme du support.

9. Implant (1) selon l'une quelconque des revendications 7 à 8, **caractérisé par** des moyens pneumatiques destinés à modifier la forme du support.

10. Implant (1) selon l'une quelconque des revendications 7 à 9, **caractérisé par** une unité de commande pour les moyens destinés à modifier la forme du support.

11. Implant (1) selon la revendication 10, **caractérisé par** une liaison amovible entre l'unité de commande et les moyens destinés à modifier la forme du support.

12. Implant (1) selon l'une des revendications 10 à 11, **caractérisé par** un conduit de communication entre l'unité de commande et l'appareil de mesure du courant (7).

13. Implant cochléaire formant l'implant (1) selon l'une quelconque des revendications 1 à 12.

14. Système formé par un implant (1), un corps creux (2) entourant ce dernier, des parties creuses du corps humain ou animal ayant été ménagées, ainsi que par un électrolyte (3), **caractérisé par** un implant (1) selon l'une quelconque des revendications 1 à 13.

15. Système selon la revendication 14, **caractérisé par** un liquide corporel, en particulier la périlymphe, formant l'électrolyte (3).

16. Procédé de réalisation d'un système selon l'une des revendications 14 à 15, comportant les étapes :
- introduction de l'implant (1) dans le corps creux (2) ;
- modification de la forme, en particulier de la courbure, de l'implant (1), de telle sorte que son extrémité avant, par référence au sens d'introduction, n'entre pas en contact avec la paroi du corps creux (2).

17. Procédé selon la revendication 16, **caractérisé en ce que** l'écartement entre l'extrémité de l'implant (1) et la paroi du corps creux (2) est mesuré.

18. Procédé selon l'une des revendications 16 à 17, **caractérisé en ce qu'**un écartement décroissant est compensé au moins partiellement par une modification de la forme de l'implant (1).

19. Procédé selon l'une quelconque des revendications 16 à 18, **caractérisé en ce que** l'écartement est mesuré selon un procédé comportant les étapes suivantes :
- application d'une tension alternative contre la masse au niveau d'au moins un point d'alimentation (4) ;
- mesure du courant entre au moins un point de mesure (5) et la masse ;
- détermination de l'écartement à partir de la dépendance entre le courant mesuré et la tension appliquée.

20. Procédé selon la revendication 19, **caractérisé par** une tension alternative avec une fréquence entre 1 kHz et 1 MHz.

21. Procédé selon l'une des revendications 19 à 20, **caractérisé par** une tension alternative avec une amplitude entre 2 mV et 10 mV.

22. Procédé selon l'une quelconque des revendications 19 à 21, **caractérisé en ce que** le système formé par l'implant (1) et le corps creux (2) est modelé sur la base d'un circuit d'équivalence avant la mesure proprement dite.

23. Procédé selon l'une quelconque des revendications 19 à 22, **caractérisé en ce qu'**au moins un point d'alimentation (4) et au moins un point de mesure (5) sont maintenus à un potentiel constant commun.

24. Procédé selon l'une quelconque des revendications 19 à 23, **caractérisé en ce que** la mesure est effectuée avec au moins deux fréquences différentes de la tension alternative appliquée.

25. Procédé selon l'une quelconque des revendications 19 à 24, **caractérisé par** plusieurs points d'alimentation (4).

26. Procédé selon l'une quelconque des revendications 19 à 25, **caractérisé par** plusieurs points de mesure (5).

27. Procédé selon l'une quelconque des revendications 19 à 26, **caractérisé par** des électrodes formant des points d'alimentation (4) et/ou des points de mesure (5).

28. Procédé selon l'une quelconque des revendications 19 à 27, **caractérisé en ce que** tous les points d'alimentation (4) et tous les points de mesure (5) sont situés sur l'implant (1).

29. Procédé selon l'une quelconque des revendications 19 à 28, **caractérisé par** des électrodes de stimulation d'un implant cochléaire pour former des points d'alimentation (4) et/ou des points de mesure (5).
